Europäisches Patentamt

European Patent Office

Office européen des brevets

(11) Veröffentlichungsnummer: **0 279 290**
A2

(12) **EUROPÄISCHE PATENTANMELDUNG**

(21) Anmeldenummer: 88101596.0

(22) Anmeldetag: 04.02.88

(51) Int. Cl.⁴: **A61K 31/44** , A61K 31/47 , A61K 31/505 , A61K 31/51 , A61K 31/35 , A23K 1/17 , //(A61K31/44,31:35), · (A61K31/47,31:35), (A61K31/505,31:35), (A61K31/51,31:35),(A61K31/35, 31:245)

Patentansprüche für folgenden Vertragsstaat: ES.

(30) Priorität: 07.02.87 DE 3703789

(43) Veröffentlichungstag der Anmeldung: 24.08.88 Patentblatt 88/34

(84) Benannte Vertragsstaaten: BE CH DE ES FR GB IT LI

(71) Anmelder: HOECHST AKTIENGESELLSCHAFT Postfach 80 03 20 D-6230 Frankfurt am Main 80(DE)

(72) Erfinder: Raether, Wolfgang, Dr. Falkensteinstrasse 6 D-6072 Dreieich(DE)

(54) **Coccidiozide Mittel.**

(57) Gegenstand der Erfindung sind Coccidiozide Mittel, die das Polyetherantibiotikum Lasalocid A oder dessen Salz oder Ester in Kombination mit einem oder mehreren Wirkstoffen der Gruppe Meticlorpindol, Methyl Benzoquat, Nicarbazin, Amprolium, Beclotiamine oder Halofuginon oder dessen Salze enthalten.

EP 0 279 290 A2

## Coccidiozide Mittel

Lasalocid A ist als Polyetherantibioticum aus US-PS 3,715,372 und DE-PS 2,040,998 bekannt, s. auch Merck Index 10. Aufl., S. 773 (1983). Dort wird auch dessen Verwendung als Anticoccidiosemittel beschrieben. Die Verbindung wird durch Fermentation hergestellt.

Es wurde nun gefunden, daß die Wirkung von Lasalocid A bei dessen Kombination mit anderen bekannten coccidioziden Wirkstoffen über das zu erwartende Maß hinaus erhöht wird.

Gegenstand der Erfindung sind daher coccidiozide Mittel, die gekennzeichnet sind durch einen Gehalt an dem Polyhetherantibiotikum Lasalocid A oder dessen physiologisch verträglichem Salz oder Ester in Kombination mit einem oder mehreren Wirkstoffen der Gruppe Meticlorpindol, Methyl Benzoquat, Nicarbazin, Amprolium, Beclotiamine oder Halofuginon oder dessen Salz.

Insbesondere kommen Zweierkombinationen in Betracht. Erfindungsgemäß können auch Dreierkombinationen verwendet werden; von den letzten ist bevorzugt die Kombination von Lasalocid A mit Meticlorpindol und Methyl Benzoquat.

Außerdem kann anstelle von Amprolium dessen Gemisch mit Ethopabate erfindungsgemäße eingesetzt werden.

Die erfindungsgemäß zu verwendenden Kombinationspartner für Lasalocid A sind in der Veterinärmedizin seit längerer Zeit bekannt. Sie werden alle im Merck Index, 10. Aufl., veröff. durch Merck & Co., Inc. USA (1983) beschrieben: Meticlorpindol (3,5-Dichlor-2,6-dimethyl-4-pyridinol) auf S. 341; Methyl Benzoquat (Nequinate; 3-Methoxycarbonyl-6-n-butyl-7-benzyloxy-4-oxochinolin auf S. 928; Nicarbazin (Komplex aus N,N'-Bis(4-nitrophenyl)harnstoff mit 4,6-Dimethyl-2(1H)-pyrimidinon auf S. 931; Ethopabate (4-Acetamido-2-ethoxy-benzoesäuremethylester) S. 544; Amprolium (1-[(4-Amino-2-propyl-5-pyrimidinyl)-methyl]-2-methyl-piperidinium-chlorid) S. 613; Beclotiamine (3-[(4-Amino-2-methyl-5-pyrimidinyl)methyl]-5-(2-chlorethyl)-4-methyl-thiazolium-chlorid) S. 144 und Halofuginon (7-Brom-6-Chlor-Febrifugine) auf S. 662. Neben Halofuginon werden erfindungsgemäßen auch deren physiologisch verträgliche Salze wie das Hydrohalogenid, insbesondere Hydrobromid, Acetat, Lactat, Alkali-oder Erdalkalisalz, Salz der Acetursäure s. DE-OS 2934069 erfaßt, sowie alle optischen Isomeren bzw. deren Gemische. Amprolium oder Beclotiamine können in Form ihrer Hydrochlorid-Additions-salze eingesetzt werden. Anstelle von Beclotiamine kann auch dessen entsprechendes Naphthalin-1,5-disulfonsäuresalz eingesetzt werden.

Die Kombination von Meticlorpindol und Methyl Benzoquat ist als Handelsprodukt.[R] Lerbek (Fa. ICI) bekannt. Die Kombination von Amprolium mit Ethopabate ist als Handelsprodukt [R] Amprol Mix Super auf dem Markt (Merck, Sharp & Dohme).

Lasalocid A kann als freie Säure, als Salz, insbesondere als Alkali-(Na, K)-, Erdalkali-(Mg,Ca)-oder Ammoniumsalz, oder als Ester, insbesondere als $(C_1-C_4)$-Alkylester oder Benzylester eingesetzt werden; bevorzugt wird das Na-Salz verwendet. Die Produkte können in gereinigter Form oder als Mycel oder Rohprodukt eingesetzt werden.

Die erfindungsgemäßen Kombinationen zeigen synergistische Effekte bei der Bekämpfung der Coccidiose, insbesondere der Geflügelcoccidiose.

Bei der Massenhaltung von Geflügel auf engem Raum, z.B. Geflügelmast oder Geflügelaufzucht, besteht stets die potentielle Gefahr einer durch Eimeria-Spezies verursachten Coccidieninfektion, die ohne chemotherapeutische Bekämpfung zu ernsten wirtschaftlichen Verlusten führt. Coccidieninfektionen verursachen in der Regel Gewichtsdepression und blutige Kotausscheidungen, die als Folge von Läsionen in der Darmschleimhaut auftreten. Schwere Coccidieninfektionen führen beim Geflügel in der Regel auch zu hoher Mortalität.

Durch die Anwendung der erfindungsgemäßen Kombinationen ergeben sich im Vergleich zu den entsprechenden Einzelwirkstoffen erhebliche Vorteile, da geringere Mengen an coccidioziden Mitteln eingesetzt werden können. Hieraus resultieren eine Reduzierung der toxischen Nebenwirkungen der Präparate im Vergleich zur Applikation der Einzelwirkstoffe, eine erhöhte Wirtschaftlichkeit sowie Verringerung der Rückstände in den eßbaren Geweben des Geflügels.

In den erfindungsgemäßen Mitteln können die Gewischtsverhältnisse der Wirkstoffe in weiten Grenzen variieren um synegistische Effekte zu erzielen, Sie liegen bei Kombinationen von Lasalocid

a) mit Meticlorpindol vorzugsweise zwischen 10:1 bis 1:6, insbesondere zwischen 4:1 bis 1:3

b) mit Methyl Benzoquat vorzugsweise zwischen 150:1 bis 1:12, insbesondere zwischen 100:1 bis 8:1

c) mit Nicarbazin vorzugsweise zwischen 10:1 und 1:6, insbesondere zwischen 4:1 und 1:2,5.

d) mit Halofuginon vorzugsweises zwischen 300:1 bis 8:1 insbesondere zwischen 166:1 bis 26:1

e) mit einem Gemisch von Meticlorpindol und Methyl Benzoquat vorzugsweise zwischen 30:1 bis 1:5, insbesondere 16:1 bis 1,3:1

f) mit Amprolium vorzugsweise zwischen 1,5:1 und 1:6

g) mit Beclotiamine vorzugsweise zwischen 4:1 und 1:5

h) mit einem Gemisch von Amprolium und Ethopabate vorzugsweise zwichen 5:1 und 1:4 (Jeweils Verhältnis Lasalocid zum Kombinationspartner)

Das Verhältnis von Meticlorpindol zu Methyl Benzoquat kann im Falle e) vorzugsweise zwischen 20:1 vus 7:1 variieren, während das Verhältnis Amprolium zu Ethopabate im Falle h)vorzugsweise zwischen 30:1 und 10.1 variiert.

Die erfindungsgemäßen Kombinationen eignen sich ganz allgemein zum Schutz von Geflügel, d.h. zur Behandlung von landwirtschaftlichem Nutzgeflügel, wie Hühner, Truthühner, Enten oder Gänsen oder auch anderen Vögeln, wie z.B. Fasanen, Wachteln oder Perlhühnern; letztere Vogelarten werden neuerdings auch in Farmen zur wirtschaftlichen Nutzung gehalten und ebenso wie Hühner häufig und massiv von Coccidien befallen.

Zum erfolgreichen Schutz des Geflügels gegen Coccidiose können die erfindungsgemäßen Mittel zu jeder Zeit eingesetzt werden. Sie können insbesondere in Masthühnerbetrieben oder Aufzuchthäusern von Junghennen Anwendung finden, da dort aufgrund der dauernd im Kot ausgeschiedenen Dauerformen der Cocccidien (Oocysten) ein hoher Infektionsdruck für die Geflügelpopulation entsteht. Da unter diesen Bedingungen stets das Risiko eines Coccidioseausbruches besteht, sollten die erfindungsgemäßen coccidioziden Mittel beim Geflügel kontinuierlich und vor Ausbruch einer Coccidiose eingesetzt werden. Die erfindungsgemäßen Mittel können aber auch während kurzer Zeitintervalle, d.h. weniger Tage verabreicht werden.

Der Einsatz der erfindungsgemäßen Mittel und Methoden zur Bekämpfung von Coccidiose wird in üblicher Weise durchgeführt. Entsprechend der Lokalisation der Coccidien im Intestinaltrakt kommt in erster Linie eine orale Applikation infrage. Die erfindunsgemäßen Wirkstoffkombinationen können dabei mit Futtermitteln oder mit Trinkwasser gemischt sein.

Die Wirkstoffkonzentrationen der Kombinationen in den Futtermitteln oder im Trinkwasser können innerhalb bestimmter Grenzen variieren. Sie liegen im allgemeinen zwischen 5 und 300 ppm der Wirkstoffkombination bezogen auf das Futtermittel bzw. Trinkwasser.

Die besonders bevorzugten Konzentrationen für die Bekämpfung der Coccidiose sind im Falle des Futtermittels jeweils 25 bis 150 ppm, insbesondere 40 bis 125 ppm Lasalocid A und jeweils

a) 15 bis 150 ppm, insbesondere 30 bis 125 ppm Meticlorpindol

b) 1 bis 30 ppm, insbesondere 1,25 bis 5 ppm Methyl Benzoquat

c) 15 bis 150 ppm, insbesondere 30 bis 100 Nicarbazin

d) 0,5 bis 3 ppm, insbesondere 0,75 bis 1,5 ppm Halofuginon und

e) 5 bis 120 ppm, insbesondere 8 bis 30 ppm Meticlorpindol/Methyl Benzoquat

f) 100 bis 150 ppm Amprolium

g) 40 bis 125 ppm Beclotiamine

h) 30 bis 100 ppm Amprolium/Ethopabat.

Im Falle der Verwendung im Trinkwasser wird bevorzugt jeweils ca. die Hälfte der angegebenen Konzentrationen benutzt.

Alle erwähnten Konzentrationen, Verhältnisse, Teile, Mengen oder Prozentangaben sind auf Gewichtseinheiten bezogen.

Die Wirkstoffkonzentrationen der coccidioziden Mittel sind auf Futter-oder Trinkwasserzubereitungen ad libitum bezogen, d.h. zur freien Futter-oder Trinkwasseraufnahme während einer praxisüblichen Mast-oder Aufzuchtperiode. Aufgrund besonderer durch die Praxis bedingter Faktoren kann es sich aber ergeben, daß der Geflügelfachmann diese Anwendungskonzentrationen nach oben anpassen muß, falls das Geflügel mit verschiedenen Futter-oder Wasservorräten versorgt werden muß. Dann enthält aber nur ein Teil der Futter-oder Wasservorräte die erfindungsgemäßen Mittel.

Als Träger die erfindungsgemäßen synergistisch wirksamen coccidioziden Mittel eignen sich alle in der Geflügelindustrie üblichen Futterformulierungen. Die im folgenden angegebenen formulierungen für Geflügelfutter sind Beispiele für praxisübliche Formulierungen. Darüber hinaus lassen sich aber auch andere Futtermittel auf der Basis von irgendwelchen Getreidekörnern verwenden, die Vitaminkonzentrate, Mineralkonzentrate oder sonstige Wirkstoffe und Futterzusätze in beliebiger Konzentration enthalten. Sowohl herkömmliche trockene, mehlige oder pelletierte Futtermittel als auch flüssige Suspensionsfutter unter Einschluß von Futtermitteln, wie Destillationsschlempen und Milchnebenprodukten, können bei den erfindungsgemäßen Mitteln verwendet werden.

Für die Herstellung des erfindungsgemäßen Geflügelfutters setzt man gewöhnlich zuerst ein konzentriertes Vorgemisch an, das die synergistisch wirksamen coccidioziden Mittel in hoher Konzentration, beispielsweise von 0,2 bis 75%, enthält. Zu diesem Zweck werden die coccidioziden Mittel mit physiologisch unbedenklichen Trägern, wie Propylenglykolen, Polyethylenglykolen, inerten Ölen, wie Pflanzenölen, hochraffinierten Mineralölen, Äthanol, Wasser, wässrigen Alkoholen, entweder dispergiert oder in inerte Trägerstoffe eingemischt, wie Vermikulit, Diatomeenerde, Attapulgit, Kalziumkarbonat, Bolus alba. Ebenso eignen sich hierfür organische Trägermaterialien, wie Weizenkleie, Maisschrot, Sojabohnenmehl, Luzernmehl, Reishülsen oder gemahlene Maiskolben und beliebig ander organische Trägerstoffe aus pflanzlichen Produkten.

Die erfindungsgemäßen synergistisch wirksamen Mittel lassen sich ferner auch zusammen mit dem Trinkwasser an Geflügel verabreichen. Ihre Einarbeitung in das Trinkwasser wird durch Zusatz einer wasserlöslichen oder in Wasser suspendierbaren Form der jeweiligen Mittel zu dem Trinkwasser in geeigneter Menge erreicht. Die Herstellung solcher Zubereitungen erfolgt im allgemeinen durch Auswahl eine wasserlöslichen Form der Mittel. Sofern diese nicht gewünscht oder nicht herstellbar sind, können auch wasser unlösliche Formen, beispielsweise Supensionen Verwendun finden. Zur Herstellung der Zubereitungen verwendet man physiologisch unbedenkliche Hilfsmittel, durch die die erfindungsgemäßen coccidioziden Mittel in Wasser über längere Zeit in Suspensionen gehalten werden. Hilfsmittel, die sich hierfür eignen sind Quellmittel, wie Alginate, Gelatine, Carboxymethylcellulose oder Polyvinylpyrrolidon. Die erfindungsgemäßen Mittel können aber auch mit verschiedenen oberflächenaktiven Verbindungen suspendiert werden, wie beispielsweise mit Hilfe von Lecithin, Naphthalinsulfonaten, Alkylbenzolsulfonaten, alkylphenol-Polyethylenoxid-Addukten oder Polyoxyethylensorbitanestern. Üblicherweise stellt man zunächst eine konzentrierte Suspension oder auch eine trockene Formulierung aus den erfindungsgemäßen coccidioziden Mitteln und den Suspendierungsmitteln in bestimmten Mischungsverhältnissen her und verdünnt sie dann mit dem Trinkwasser auf die gewünschte Anwendungskonzentration, oder die Vormischungen werden in geeigneten Konzentrationen mit den in der Praxis üblichen Futtermitteln gemischt. Das so medikierte Trinkwasser bzw. Futter wird dann dem Geflügel entweder zunächst ad libitum oder eine bestimmte Zeit angeboten.

Das erfindungsgemäße Behandlungsverfahren läßt sich auch auf andere Verfahren zur Behandlung und Fütterung von Geflügel erweitern. So können beispielsweise die erfindungsgemäßen Zubereitungen mit anderen Wirkstoffen kombiniert werden, wie z.B. mit wachstumsfördernden Mitteln oder Parasitika, die einerseits synthetische Mittel oder andererseits Fermentationsprodukte im weitesten Sinn darstellen.

Die Erfindung ist zwar im besonderen Maße auf den Schutz des Geflügels gegenüber Coccidieninfektionen gerichtet, ist jedoch auch sinngemäß bei anderen Haus-und Nutztieren, wie beispielsweise anderen Vögeln, Kaninchen, Schweinen und Wiederkäuern anzuwenden.

Gegenstand der Erfindung ist daher auch ein Verfahren zur Behandlung der Geflügelcoccidiose, das darin besteht, daß man obengenannte wriksame Mittel oral an Geflügel ad libitum verabreicht, und zwar entweder als Futtermittel oder mittels Trinkwasser.

Die Erfindung wird durch nachfolgende Beispiele erläutert.

## A. Beispiels für Tierfutter-Zusammensetzungen

Die folgenden Beispiele beziehen sich auf Tierfutterzusammensetzungen, welche zur Applikation der erfindungsgemäßen Wirkstoffkombination benutzt werden können.

I. Mastfutter für Broiler

Zusammensetzung in Gew.-%

| | |
|---|---|
| Fischmehl (60 - 65 %) | 6,0 |
| Futterhefe | 2,0 |
| Rindertalg | 4,7 |
| Sojaschrot (44 %) | 24,0 |
| Luzernegrünmehl | 1,0 |
| Mais | 44,89 |
| Weizen | 6,35 |
| Weizennachmehl | 8,5 |
| Phosphors. Futterkalk | 1,4 |
| Kohlens. Futterkalk | 0,96 |
| Spurenelementvormischung* | 0,04 |
| Viehsalz | 0,09 |
| Methionin DL | 0,07 |
| | 100,0 |

a) pro kg Futter werden zugesetzt:

| | | |
|---|---|---|
| Vitamin A | i.E. | 12.000 |
| $D_3$ | i.E. | 1.500 |
| E | mg | 18 |
| $B_1$ | mg | 1,5 |
| $B_2$ | mg | 6 |
| Pantothensäure | mg | 9 |
| Nicotinsäure | mg | 24 |
| Vitamin $B_6$ | mg | 4,5 |
| $B_{12}$ | mcg | 24 |
| $K_3$ | mg | 3 |
| Cholinchlorid | mg | 1.300 |

*b) In 1,0 kg Futter enthalten

| | | |
|---|---|---|
| Mn | mg | 106 |
| Zn | mg | 71 |
| Fe | mg | 44 |
| Cu | mg | 3,56 |
| I | mg | 0,4 |
| Co | mg | 0,16 |

II. <u>Aufzuchtfutter</u> <u>für</u> <u>Küken</u> (0. - 8.Woche)

<u>Zusammensetzung in Gew.-%</u>

| | |
|---|---|
| Fischmehl (60 - 65 %) | 5,0 |
| Luzernegrünmehl | 6,0 |
| Sojaschrot (44 %) | 13,0 |
| Futterhefe | 2,8 |
| Rindertalg | 2,0 |
| Gerste | 6,0 |
| Hafer | 6,0 |
| Mais | 37,0 |
| Weizen | 13,0 |
| Weizenkleie | 7,3 |
| Kohlens. Futterkalk | 1,29 |
| (R)Hostaphos | 0,57 |
| Spurenelementvormischung * | 0,04 |
| | 100,0 |

* s. Beispiel I b); ferner werden , wie in Beispiel I a) beschrieben, Vitamine zugesetzt.

III. Aufzuchtfutter für Küken (9. - 20. Woche)

<u>Zusammensetzung in Gew.-%</u>

| | |
|---|---|
| Luzernegrünmehl | 6,0 |
| Sojaschrot (44 %) | 10,24 |
| Futterhefe | 1,8 |
| Rindertalg | 2,0 |
| Gerste | 8,0 |
| Hafer | 6,0 |
| Mais | 40,0 |
| Weizen | 15,0 |
| Weizenkleie | 8,3 |
| Kohlens. Futterkalk | 1,53 |
| (R)Hostaphos | 1,02 |
| Spurenelementvormischung * | 0,04 |
| Methionin DL | 0,07 |
| | 100,0 |

* s. Beispiel I b); ferner werden Vitamine zugesetzt, s. Beispiel I a)

IV. Aufzuchtfutter für Küken (ab 21. Woche)

<u>Zusammensetzung in Gew.-%</u>

| | |
|---|---|
| Fischmehl (60 - 65 %) | 1,5 |
| Sojaschrot (44 %) | 19,31 |
| Rindertalg | 1,0 |
| Hafer | 6,7 |
| Mais | 40,0 |
| Weizen | 18,0 |
| Weizennachmehl | 3,0 |
| Methionin DL | 0,07 |
| Futterpaprika | 0,3 |
| Kohlens. Futterkalk | 8,16 |
| (R)Hostaphos | 1,91 |
| Spurenelementvormischung * | 0,05 |
| | 100,0 |

Ferner werden Vitamine, wie in Beispiel I a) beschrieben zugegeben.

V. <u>Futter</u> <u>für</u> <u>Puten</u> (Zusammensetzung in Gew.-%)

| | Putenstarter-futter 0.-8. Woche | Putenmast-futter I 9.-12. W. | Putenmast-futter II 13.-16. W. |
|---|---|---|---|
| Fischmehl | 9,0 | 5,0 | 2,0 |
| Futterhefe | 4,5 | 2,91 | 4,0 |
| Fett | | 3,1 | 6,7 |
| Sojaschrot | 26,0 | 23,9 | 23,87 |
| Luzernegrünmehl | 4,6 | 2,0 | 0,95 |
| Gerste | 5,3 | 4,5 | 5,0 |
| Hafer | | 1,0 | 4,45 |
| Mais | 36,04 | 40,02 | 39,90 |
| Weizen | 5,3 | 6,0 | 5,0 |
| Weizennachmehl | 5,95 | 4,0 | 3,8 |
| Weizenkleie | | 4,0 | |
| Phosphors. Futterkalk | 1,58 | 1,30 | 2,71 |
| Kohlens. Futterkalk | 1,35 | 1,29 | 0,96 |
| Spurenelementvormischung * | 0,04 | 0,04 | 0,04 |
| Viehsalz | 0,24 | 0,84 | 0,44 |
| Methionin | 0,1 | 0,1 | 0,18 |
| | 100,0 | 100,0 | 100,0 |

## Vitaminmischung pro kg Futter:

| | | | | |
|---|---|---|---|---|
| Vitamin A | i.E. | 12.000 | 8.000 | 8.000 |
| $D_3$ | i.E. | 1.500 | 1.000 | 1.000 |
| E | mg | 18 | 12 | 12 |
| $B_1$ | mg | 1,5 | 1 | 1 |
| $B_2$ | mg | 6,0 | 4 | 4 |
| Pantothensäure | mg | 9,0 | 6 | 6 |
| Nicotinsäure | mg | 24,0 | 16 | 16 |
| Vitamin $B_6$ | mg | 4,5 | 3 | 3 |
| $B_{12}$ | mcg | 24,0 | 16 | 16 |
| $K_3$ | mg | 3,0 | 2 | 2 |
| Cholinchlorid | mg | 1.300 | 1.300 | 1.300 |

* in einem 1 kg Futter enthalten: 106 mg Mn; 71 mg Zn; 44 mg Fe; 3,56 mg Cu; 0,4 mg I; 0,16 mg Co.

## B. Biologische Beispiele

Der coccidiostatische Effekt der vorliegenden Mittel wurde an mit Coccidien infizierten Hühnchen untersucht. Die im folgenden aufgeführten experimentellen Untersuchungen zeigen die Wirksamkeit der verschiedenen erfindungsgemäßen Kombinationen anhand einiger Beispiele. Das Salinomycin wird hierbei in Form eines Natriumsalzes des nicht aufgetrennten und durch Fermentation hergestellten Mycels eingesetzt.

Zur Bestimmung und Bewertung des coccidiostatischen Effektes der erfindungsgemäßen Mittel wurden bei allen folgenden beschriebenen experimentallen Untersuchungen sogenannte Infektionskontrollen (unbehandelte, infizierte Tiere) und 0-Kontrollen (unbehandelte, nicht infizierte Tiere) verwandt. Die hierfür verwendeten Tiere beiderlei Geschlechts (LSL Hühnchen, Fa. Lohmann, Wallau, BRD) wurden randomisiert und jeweils Gruppen von 16 Tieren zusammengestellt. Nach der gleichen Methode wurden die mit den erfindungsgemäßen Mitteln behandelten und infizierten Gruppen zusammengestellt. Die Tiere, ausgenommen diejenigen der 0-Kontrolle, wurden mit einem virulenten Eimeria tenella-Stamm infiziert, der in 8 Tage alten Hühnchen zu starken (reproduzierbaren) Läsionen der Blinddärme führt. Die coccidioziden Mittel wurden mit dem Futter für Geflügel in ppm-Mengen vermischt; die jeweiligen Konzentrationen sind aus den folgenden Tabellen 1a bis 1e zu ersehen.

Der Grad der durch die Infektion hervorgerufenen pathologisch-anatomischen Veränderungen der Darmschleimhaut in den Blinddärmen wird üblicherweise (Experimental Parasitology Vol. 28, 1970; oder Long, P.L.: The Biology of the Coccidia, 1982, Univ. Park Press) in Form von Schädigungswerten (=Läsionswerten) im folgen "lesion scores" genannt, (Skala von 0 bis 4) ausgedrückt. Am Ende der Untersuchungen wurden die Tiere 5 Tage nach der Infektion geötet und alle auf typische durch Coccidiose verursachte pathologisch-anatomischen Veränderungen untersucht.

Beschreibung und Bewertung der Schädigung (lesion scores): 0 bis 4

0 = Tiere, bei denen keine Läsionen im Intestinaltrakt nachweisbar sind

1 = Tiere mit wenigen, umschriebenen kleinen Läsionen (Petechien) in den Blinddärmen

2 = Tiere mit mehreren, umschriebenen und etwas größeren Läsionen (Petechien) als unter 1 beschrieben

3 = Tiere mit zahlreichen und relativ großflächigen, blutigen Läsionen, die teilweise konfluieren.

4 = Tiere mit großflächigen, blutigen Läsionen, die die gesamte Darmschleimhaut der Blinddärme und auch angrenzende Darmabschnitte (Hüftdarm = Ileum bzw. Mastdarm) erfaßt. Es bietet sich das Bild einer großflächigen hämorrhagischen Enteritis schwersten Grades, die in der Regel zum Tod des Tieres führt.

Bei den unter 1 bis 4 angegebenen Schädigungen wird in zunehmendem Maß dünnflüssiger und blutiger Kot abgesetzt. Die Gewichtsdepression infolge Nahrungsverweigerung korreliert ebenso mit der Zunahme der "lesion scores".

Aus den erwähnten Beobachtungen wird die Relevanz der lesion scores für die Beurteilung der coccidioziden Wirkung der erfindungsgemäßen Mittel deutlich.

Die lesionscores werden einmal als Einzelwerte pro Tier innerhalb einer Gruppe, zum anderen als Mittelwerte der entsprechenden Tiergruppe in den Tabelle 1a - 1g aufgeführt.

Behandlung und Infektionen

In allen Versuchen wurden jeweils 16 eine Woche alte LSL-Hühnchen pro Gruppe unter konstanten Raumbedingungen in Drahtkäfigen zu jeweils 4 Tieren pro Drahtkäfig gehalten. Das mit den erfindungsgemäßen Mitteln medikierte Futter wurde vom Tage D-1 (ein Tag vor der Infektion) bis zum Tage D + 5 (5 Tage nach der Infektion) ad libitum angeboten. Die Kontrollgruppen (0-Gruppe bzw. Infektionskontrolle) erhielten nicht medikiertes Futter. Die jeweiligen Tiergruppen wurden somit 7 Tage mit dem gleichen Futter ge füttert. Am Tag der Infektion ( = D0) wurden je Tier 200.000 sporulierte Oocysten obengenannten E. tenella-Stammes per Schlundsonde appliziert.

Nach Andauung der Oocysten im Dünndarm wurden aus diesen Dauerformen die infektiösen Sporozoiten freigesetzt, die anschließend die Epithelzellen der Darmschleimhaut befallen und sich dort massenhaft durch Formenänderung vermehren. Die aus den Sporozoiten SICH entwickelnden zahlreichen Schizonten zerstören durch wiederholte Teilungsvorgänge das Darmepithel. Der Höhepunkt des zerstörenden Effektes der Schizonten auf das Darmepithel ist 5 Tage nach der Infektion erreicht. Deshalb wurden zu diesem Zeitpunkt der oben beschriebenen Weise die "lesion scores" ermittelt.

Der synergistische Effekt der erfindungsgemäßen Mittel (Kombinationen) wird im folgenden in den Tabellen 1a - 1g dargestellt, und zwar einmal der Effekt der Einzelwirkstoffe in der praxisüblichen Anwendungskonzentration und zum anderen der aus verschiedenen Kombinatinen der Einzelwirkstoffe resultierende überadditive oder synergistische Effekt. Die "lesion scores" der Infektionskontrollen bzw. der 0-Kontrollen (nicht infizierte Tiere) dienen der Abgleichung mit den jeweiligen medikierten und infizierten Tiergruppen.

## Tabelle 1a

### Kombinationen von Lasalocid A mit Meticlorpindol

| Behandlung (medikiertes Futter) | Konzentration ppm | Schädigung Einzelwert pro Tier | (lesion scores) Summe | Mittelwert pro Gruppe |
|---|---|---|---|---|
| keine (Infektionskontrolle) | 0 | 3 3 3 3 3 4 4 4<br>4 4 4 4 4 4 4 4 | 59 | 3,7 |
| keine (nicht infiz. Kontrolle) | 0 | 0 0 0 0 0 0 0 0<br>0 0 0 0 0 0 0 0 | 0 | 0 |
| Lasalocid A | 125 | 0 0 0 0 0 0 0 0<br>0 0 0 0 0 0 0 2 | 2 | 0,1 |
| | 62,5 | 0 1 1 1 1 1 2 2<br>3 3 3 3 3 3 3 4 | 34 | 2,1 |
| Meticlorpindol | 125 | 0 0 0 0 0 0 0 0<br>0 0 0 0 0 0 3 3 | 6 | 0,4 |
| | 62,5 | 0 0 0 0 0 0 1 2<br>2 3 3 3 4 4 4 4 | 30 | 1,9 |
| | 31,25 | 3 3 3 3 3 3 3 3<br>3 3 3 3 3 3 4 4 | 50 | 3,1 |
| Lasalocid A + Meticlorpindol | 62,5+62,5 | 0 0 0 0 0 0 0 0<br>0 0 0 0 0 0 0 0 | 0 | 0 |
| | 62,5+31,25 | 0 0 0 0 0 0 0 0<br>0 0 0 0 0 0 0 0 | 0 | 0 |

0 279 290

Tabelle 1b

### Kombinationen von Lasalocid A mit Methylbenzoquate

| Behandlung (medikiertes Futter) | Konzentration ppm | Schädigung (lesion scores) Einzelwert pro Tier | Summe | Mittelwert pro Gruppe |
|---|---|---|---|---|
| keine (Infektionskontrolle) | 0 | 3 3 3 3 3 4 4 4<br>4 4 4 4 4 4 4 4 | 59 | 3,7 |
| keine (nicht infiz. Kontrolle) | 0 | 0 0 0 0 0 0 0 0<br>0 0 0 0 0 0 0 0 | 0 | 0 |
| Lasalocid A | 125 | 0 0 0 0 0 0 0 0<br>0 0 0 0 0 0 0 2 | 2 | 0,1 |
| | 62,5 | 0 1 1 1 1 1 2 2<br>3 3 3 3 3 3 3 4 | 34 | 2,1 |
| | 31,25 | 3 3 3 3 3 3 3 3<br>3 3 3 4 4 4 4 4 | 53 | 3,3 |
| Methylbenzoquate | 10 | 0 0 0 0 0 0 0 0<br>0 0 0 0 0 0 0 0 | 0 | 0 |
| | 2,5 | 0 1 2 3 3 3 3 3<br>3 3 3 4 4 4 4 4 | 47 | 2,9 |
| Lasalocid A + Methylbenzoquate | 62,5+2,5 | 0 0 0 0 0 0 0 0<br>0 0 0 0 0 0 0 0 | 0 | 0 |
| | 31,25+2,5 | 0 0 0 0 0 0 0 0<br>0 1 1 1 3 3 3 3 | 15 | 0,9 |

## Tabelle 1c

### Kombinationen von Lasalocid A mit Nicarbazin

| Behandlung (medikiertes Futter) | Konzentration ppm | Schädigung (lesion scores) | | Mittelwert pro Gruppe |
|---|---|---|---|---|
| | | Einzelwert pro Tier | Summe | |
| keine (Infektionskontrolle) | 0 | 3 3 3 3 3 3 4<br>4 4 4 4 4 4 4 4 | 57 | 3,6 |
| keine (nicht infiz. Kontrolle) | 0 | 0 0 0 0 0 0 0 0<br>0 0 0 0 0 0 0 0 | 0 | 0 |
| Lasalocid A | 125 | 0 0 0 0 0 0 0 0<br>0 0 0 0 0 0 1 2 | 3 | 0,2 |
| | 62,5 | 0 0 1 1 1 3 3 3<br>3 3 3 4 4 4 4 4 | 41 | 2,6 |
| Nicarbazin | 125 | 0 0 0 0 0 0 0 0<br>1 3 3 3 3 3 3 3 | 22 | 1,4 |
| | 62,5 | 1 1 1 3 3 3 3 3<br>3 4 4 4 4 4 4 4 | 49 | 3,1 |
| | 31,25 | 1 3 3 3 3 3 3 3<br>4 4 4 4 4 4 4 4 | 54 | 3,4 |
| Lasalocid A + Nicarbazin | 62,5+62,5 | 0 0 0 0 0 0 0 0<br>0 0 0 0 0 0 0 0 | 0 | 0 |
| | 61,2+31,25 | 0 0 0 0 0 0 0 0<br>0 0 0 0 0 1 2 3 | 6 | 0,4 |

0 279 290

## Tabelle 1d

### Kombinationen von Lasalocid A mit Halofuginone

| Behandlung (medikiertes Futter) | Konzentration ppm | Schädigung (lesion scores) Einzelwert pro Tier | Summe | Mittelwert pro Gruppe |
|---|---|---|---|---|
| keine (Infektionskontrolle) | 0 | 3 3 3 3 3 3 3 4<br>4 4 4 4 4 4 4 4 | 57 | 3,6 |
| keine (nicht infiz. Kontrolle) | 0 | 0 0 0 0 0 0 0 0<br>0 0 0 0 0 0 0 0 | 0 | 0 |
| Lasalocid A | 125 | 0 0 0 0 0 0 0 0<br>0 0 0 0 0 0 1 2 | 3 | 0,2 |
|  | 62,5 | 0 0 1 1 1 3 3 3<br>3 3 3 4 4 4 4 4 | 41 | 2,6 |
| Halofuginone | 3 | 0 0 0 0 0 0 0 0<br>0 0 0 0 0 0 0 0 | 0 | 0 |
|  | 1,5 | 0 0 0 0 0 0 0 0<br>0 1 1 1 3 3 3 3 | 15 | 0,9 |
|  | 0,75 | 1 1 1 1 1 3 3 3<br>3 3 3 4 4 4 4 4 | 43 | 2,7 |
| Lasalocid A + Halofuginone | 62,5+1,5 | 0 0 0 0 0 0 0 0<br>0 0 0 0 0 0 0 0 | 0 | 0 |
|  | 62,5+0,75 | 0 0 0 0 0 0 0 0<br>0 0 0 0 0 0 1 3 | 4 | 0,3 |

## Tabelle 1e

### Kombinationen von Lasalocid A mit Meticlorpindol/Methylbenzoquate

| Behandlung (medikiertes Futter) | Konzentration ppm | Schädigung (lesion scores) Einzelwert pro Tier | Summe | Mittelwert pro Gruppe |
|---|---|---|---|---|
| keine (Infektionskontrolle) | 0 | 2 3 3 3 4 4 4<br>4 4 4 4 4 4 4 4 | 58 | 3,6 |
| keine (nicht infiz. Kontrolle) | 0 | 0 0 0 0 0 0 0 0<br>0 0 0 0 0 0 0 0 | 0 | 0 |
| Lasalocid A | 125 | 0 0 0 0 0 0 0 0<br>0 0 0 0 1 1 2 3 | 7 | 0,4 |
| | 62,5 | 0 1 1 1 1 1 3 3<br>3 4 4 4 4 4 4 4 | 42 | 2,6 |
| | 108,35 | 0 0 0 0 0 0 0 0<br>0 0 0 0 0 0 0 0 | 0 | 0 |
| Meticlorpindol/ Methylbenzoquate (100 Teile/9,35 Teile) | 9,5 | 0 0 1 1 1 1 1 1<br>2 3 3 3 3 3 3 3 | 29 | 1,8 |
| | 0,75 | 0 1 3 3 3 3 3 3<br>4 4 4 4 4 4 4 4 | 51 | 3,2 |
| Lasalocid A + Meticlorpindol/ Methylbenzoquate | 62,5+9,5 | 0 0 0 0 0 0 0 0<br>0 0 0 0 0 0 0 0 | 0 | 0 |
| | 62,5+6,75 | 0 0 0 0 0 0 0 0<br>0 0 0 0 1 1 3 3 | 8 | 0,5 |

## Tabelle 1f

### Kombinationen von Lasalocid A mit Amprolium

| Behandlung (medikiertes Futter) | Konzentration ppm | Schädigung Einzelwert pro Tier | (lesion scores) Summe | Mittelwert pro Gruppe |
|---|---|---|---|---|
| keine (Infektionskontrolle) | 0 | 2 2 3 3 3 3 3 3<br>4 4 4 4 4 4 4 4 | 54 | 3,4 |
| keine (nicht infiz. Kontrolle) | 0 | 0 0 0 0 0 0 0 0<br>0 0 0 0 0 0 0 0 | 0 | 0 |
| Lasalocid A | 125 | 0 0 0 0 0 0 0 0<br>0 0 0 0 1 1 2 3 | 3 | 0,2 |
| | 62,5 | 0 1 1 1 1 1 1 1<br>2 3 3 3 3 3 4 4 | 32 | 2,0 |
| Amprolium | 125 | 0 0 0 0 0 0 0 0<br>0 0 0 0 0 0 0 0 | 0 | 0 |
| | 62,5 | 0 0 0 0 0 0 0 1<br>1 1 1 1 1 2 2 2 | 12 | 0,8 |
| | 31,25 | 0 0 0 1 1 1 1 2<br>2 3 3 3 3 3 3 4 | 30 | 1,9 |
| Lasalocid A + Amprolium | 62,5+62,5 | 0 0 0 0 0 0 0 0<br>0 0 0 0 0 0 0 0 | 0 | 0 |
| | 62,5+31,25 | 0 0 0 0 0 0 0 0<br>0 0 0 0 1 1 3 3 | 1 | 0,1 |

0 279 290

## Tabelle 1g

### Kombinationen von Lasalocid A mit Amprolium + Ethopabate

| Behandlung (medikiertes Futter) | Konzentration ppm | Schädigung Einzelwert pro Tier | (lesion scores) Summe | Mittelwert pro Gruppe |
|---|---|---|---|---|
| keine (Infektionskontrolle) | 0 | 2 2 3 3 3 3 3 3<br>4 4 4 4 4 4 4 4 | 54 | 3,4 |
| keine (nicht infiz. Kontrolle) | 0 | 0 0 0 0 0 0 0 0<br>0 0 0 0 0 0 0 0 | 0 | 0 |
| Lasalocid A | 125 | 0 0 0 0 0 0 0 0<br>0 0 0 0 1 1 2 3 | 3 | 0,2 |
| | 62,5 | 0 1 1 1 1 1 1 1<br>2 3 3 3 3 3 4 4 | 32 | 2,0 |
| Amprolium + Ethopabate (100 Teile + 6,4 Teile) | 125 | 0 0 0 0 0 0 0 0<br>0 0 0 0 0 0 0 0 | 0 | 0 |
| | 62,5 | 0 0 0 0 0 0 0 0<br>0 1 1 1 1 2 2 3 | 11 | 0,7 |
| | 31,25 | 0 0 0 0 0 1 1 1<br>1 2 2 2 2 2 3 4 | 21 | 1,3 |
| Lasalocid A + Amprolium + Ethopabate | 62,5+62,5 | 0 0 0 0 0 0 0 0<br>0 0 0 0 0 0 0 0 | 0 | 0 |
| | 62,5+31,25 | 0 0 0 0 0 0 0 0<br>0 0 0 0 0 0 0 0 | 0 | 0 |

0 279 290

## Ansprüche

1. Coccidiozode Mittel, gekennzeichnet durch einen Gehalt an dem Polyetherantibiotikum Lasalocid A oder dessen physiologisch verträglichen Salze oder Ester in Kombination mit einem oder mehreren Wirkstoffen der Gruppe Meticlorpindol, Methyl Benzoquat, Nicarbazin, Amprolium, Beclotiamine oder Halofuginon oder dessen Salz.

2. Mittel gemäß Anspruch 1, dadurch gekennzeichnet, daß es Lasalocid A in Form seiner physiologisch verträglichen Salze oder Ester enthält. .

3. Mittel nach Anspruch 2, dadurch gekennzeichnet, daß es als Salze Alkali-oder Erdalkalisalze enthält.

4. Mittel nach Ansprüchen 2 oder 3, dadurch gekennzeichnet, daß es Lasalocid A als Natrium-, Kalium-, Ammoniumsalz, Magnesium-oder Calciumsalz enthält.

5. Mittel nach einem oder mehreren der Ansprüche 1 bis 4, dadurch gekennzeichnet, daß es Lasalocid A als Na-Salz enthält.

6. Mittel nach Anspruch 1, dadurch gekennzeichnet, daß es Lasalocid A als Mycel oder Rohpodukt enthält.

7. Mittel gemäß einem oder mehreren der Ansprüche 1 bis 6, dadurch gekennzeichnet, daß es Lasalocid A in Kombination mit Meticlorpindol und Methyl Benzoquat enthält.

8. Mittel gemäß einem oder mehreren der Ansprüche 1 bis 6, dadurch gekennzeichnet, daß im Falle der Verwendung von Amprolium als Kombinationspartner zusätzlich der Wirkstoff Ethopabate enthalten ist.

9. Mittel gemäß einem oder mehreren der Ansprüche 1 bis 8, dadurch gekennzeichnet, daß es zusätzlich Geflügelfutter oder Trinkwasser enthält.

Patentansprüche für den folgenden Vertragsstaat: ES

1. Verfahren zur Herstellung eines coccidioziden Mittels, das das Polyetherantibiotikum Lasalocid A oder dessen physiologisch verträgliches Salz oder Ester in Kombination mit einem oder mehreren Wirkstoffen der Gruppe Meticlorpindol, Methyl Benzoquat, Nicarbazin, Amprolium, Beclotiamine oder Halofuginon oder dessen Salz enthält, dadurch gekennzeichnet, daß man die Wirkstoffkombinationen in geeigneter Form mit einem Futtermittel oder Trinkwasser oder galenischen Hilfsund Zusatzstoffen mischt und gegebenenfalls in eine für die orale Verabreichung geeignete Applikationsform bringt.

2. Verfahren gemäß Anspruch 1, dadurch gekennzeichnet, daß das Mittel Lasalocid A in Form seiner physiologisch verträglichen Salze oder Ester enthält.

3. Verfahren nach Anspruch 2, dadurch gekennzeichnet, daß das Mittel als Salze Alkali-oder Erdalkalisalze enthält.

4. Verfahren nach Ansprüchen 2 oder 3, dadurch gekennzeichnet, daß das Mittel Lasalocid A als Natrium-, Kalium-, Ammoniumsalz, Magensium-oder Calciumsalz enthält.

5. Verfahren nach einem oder mehreren der Ansprüche 1 bis 4, dadurch gekennzeichnet, daß das Mittel Lasalocid A als Na-Salz enthält.

6. Verfahren nach Anpsruch 1, dadurch gekennzeichnet, daß das Mittel Lasalocid A als Mycel oder Rohprodukt enthält.

7. Verfahren gemäß einem oder mehreren der Ansprüche 1 bis 6, dadurch gekennzeichnet, daß das Mittel Lasalocid A in Kombination mit Meticlorpindol und Methyl Benzoquat enthält.

8. Verfahren gemäß einem oder mehreren der Ansprüche 1 bis 6, dadurch gekennzeichnet, daß im Falle der Verwendung von Amprolium als Kombinationspartner zusätzlich der Wirkstoff Ethopabate enthalten ist.

9. Verfahren gemäß einem oder mehreren der Ansprüche 1 bis 8, dadurch gekennzeichnet, daß das Mittel Geflügelfutter oder Trinkwasser enthält.